# EUROPEAN PATENT SPECIFICATION

(11) **EP 3 613 746 B1**
(45) Date of publication and mention of the grant of the patent: **05.01.2022**
(21) Application number: 18783851.1
(22) Date of filing: 03.04.2018
(51) Int. Cl.: C07D 491/048, A61K 31/5025, A61K 31/7068, A61P 35/00

(54) **COMPOUND OF EOC315 MOD.I CRYSTAL FORM AND PREPARATION METHOD THEREFOR**
VERBINDUNG DER KRISTALLINEN FORM EOC315 MOD.I UND HERSTELLUNGSVERFAHREN DAFÜR
COMPOSÉ DE FORME CRISTALLINE MOD.I D'EOC315 ET PROCÉDÉ DE PRÉPARATION ASSOCIÉ

(30) Priority: 13.04.2017 CN 201710239073
(43) Date of publication of application: 26.02.2020
(73) Proprietor: Taizhou Eoc Pharma Co., Ltd., Taizhou Jiangsu 225300 (CN)
(72) Inventor: LI, Heting, Taizhou Jiangsu 225300 (CN); WANG, Deqiang, Taizhou Jiangsu 225300 (CN); CHANG, Wei, Taizhou Jiangsu 225300 (CN); YU, Hongrui, Taizhou Jiangsu 225300 (CN); ZOU, Xiaoming, Westlake Village, California 91361 (US)
(74) Representative: Weickmann & Weickmann PartmbB
(86) International application number: PCT/CN2018/081660
(87) International publication number: WO 2018/188495

(56) References cited:
- WO-A2-01/23375
- CN-A- 104 804 008
- CN-A- 107 129 502
- CN-B- 104 804 008
- M Alsina ET AL: "Clinical and pharmacodynamic results of TEL0805 trial", , 20 May 2011 (2011-05-20), XP055745205, DOI: 10.1200/jco.2011.29.15_suppl.4122 Retrieved from the Internet: URL:https://ascopubs.org/doi/abs/10.1200/j co.2011.29.15_suppl.4122 [retrieved on 2020-10-29]
- STRUMBERG, D et al.: "Phase I dose Escalation Study of Telatinib (BAY 57-9352) in Patients with Advanced Solid Tumours", British Journal of Cancer, vol. 99, no. 10, 31 December 2008 (2008-12-31), pages 1579-1585, XP055542364,

## Description

### TECHNICAL FIELD

The present invention belongs to the technical field of pharmaceuticals, and particularly relates to the preparation and characterization of crystalline form Mod. I of EOC315, as well as use of the crystalline form Mod. I of EOC315 in the preparation of a medicament.

### BACKGROUND

EOC315 (4-(4-chloroanilino)-7-(2-methylaminocarbonyl-4-oxymethyl) pyridylfuro[2,3-*d*]pyridazine mesylate) is not in the market yet in any country. The American biopharmaceutical company, ACT Biotech, Inc. (ACT Biotech, and ACT for short hereinafter) obtained a license for EOC315 from Bayer HealthCare Pharmaceuticals in 2008, and in January, 2014, Eddingpharm had an asset purchase agreement with ACT and obtained the global exclusive right thereof. Its structural formula is as shown below:

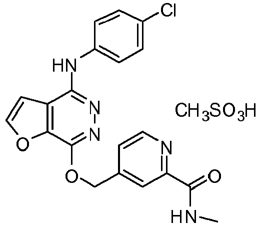

EOC315 has oral bioavailability and is a potent inhibitor of the VEGFR-2 (Vascular Endothelial Growth Factor Receptor-2) tyrosine kinase, with an IC50 value of 6 nM according to biochemical measurement. EOC315 also inhibits the activity of the PDGFR (Platelet-Derived Growth Factor Receptor) tyrosine kinase, with an IC50 value of 15 nM. These two types of receptors play a critical role in the angiogenesis process involving stimulating endothelial cells to form blood vessels, as well as in the process of PDGFR expressing pericytes supporting newly formed blood vessels. EOC315 is under development and is the most selective VEGFR inhibitor known, which can distinguish the aforementioned targets from other kinases. As an oral preparation, patients would be able to continuously inhibit said desired target in the long term.

Internationally, EOC315 is under phase II clinical development, which may be used as an anticancer drug in the chemotherapy for untreated advanced gastric cancer in combination with capecitabine/cisplatinum. So far, seven phase I, Ib and II clinical studies for EOC315 have been conducted in more than 300 patients in total.

In multiple clinical studies, the safety and efficacy of EOC315 in patients with a variety of advanced solid tumors were explored. The study results showed that EOC315 (monotherapy and combination therapy) had a good tolerance, and exhibited a good anti-tumor prospect. In particular, the efficacy and safety of EOC315 combined with capecitabine and cisplatinum as first-line treatment for patients with advanced gastric cancers or gastroesophageal junction (GEJ) cancers were evaluated in a phase II clinical study. The results showed that the overall response rate (ORR) reached 67%, which was almost twice of the historical response rate achieved with chemotherapy alone. Median progression-free survival (PFS) was 4.5 months. Kaplan-Meier analysis showed that the proportion of patients without tumor-progression within 6 months was 52%. The median time to progression for patients showing tumor remission was 6.4 months. Therefore, the current clinical development is intended for treating gastric cancer or gastroesophageal junction cancer.

A synthesis method for a compound EOC315 and analogues thereof, a pharmaceutical composition thereof, and a pharmaceutical use of the pharmaceutical composition are disclosed in patents CN00816369 and CN200510127109 of Bayer Company. A preparation method of EOC315 suitable for industrial production is also disclosed in the patent ZL200510140054. CN104804008 discloses EOC315 in crystalline form. However, the crystalline form Mod. I of EOC315 and its pharmaceutical use is disclosed for the first time in the present invention.

### SUMMARY

One of the purposes of the present invention is to provide and characterize a stable crystalline form Mod. I of EOC315.

Another purpose of the present invention is to provide a preparation method for such crystalline form.

Another purpose of the present invention is to compare changes of X-ray powder diffraction peak relative intensities, DSC and crystalline structures of EOC315 before and after micronization.

Another purpose of the present invention is to demonstrate that the crystalline form of EOC315 would not be changed by micronization, and is still the crystalline form Mod. I.

A further purpose of the present invention is to provide a pharmaceutical composition containing EOC315 Mod. I.

Another purpose of the present invention is to provide a use of the EOC315 crystalline form Mod. I in the preparation of a VEGFR-2 (Vascular Endothelial Growth Factor Receptor-2) and PDGFR (Platelet-Derived Growth Factor Receptor) kinase inhibitor.

The technical solution of the present invention is as follows:

A crystalline form Mod. I of the present invention of EOC315 as shown in Formula I:

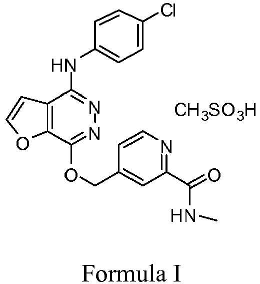

When analyzed with X-ray diffraction (CuKa), X-ray diffraction peaks appear at 4.01, 7.85, 9.94, 13.04, 19.08, 19.46, 20.10, 21.82, 22.49, 23.76, 24.26, 27.17, 28.52, 30.48 in the X-ray powder diffraction pattern.

2Θ and d(A) of the X-ray diffraction pattern are as shown in table 1.

**Table 1: 2Θ and d(A) values for the EOC315 Mod. I crystalline form of the present invention**

| 2Θ | d(A) | 2Θ | d(A) | 2Θ | d(A) |
|---|---|---|---|---|---|
| 4.006 | 22.04 | 24.265 | 3.665 | 37.04 | 2.425 |
| 7.849 | 11.255 | 24.679 | 3.604 | 37.50 | 2.396 |
| 9.936 | 8.895 | 26.201 | 3.398 | 38.519 | 2.335 |
| 11.718 | 7.546 | 27.17 | 3.280 | 39.30 | 2.291 |
| 15.861 | 5.583 | 27.61 | 3.228 | 40.00 | 2.252 |
| 17.791 | 4.981 | 28.658 | 3.112 | 41.30 | 2.184 |
| 19.084 | 4.647 | 29.828 | 2.993 | 42.43 | 2.129 |
| 19.463 | 4.557 | 30.519 | 2.923 | 43.39 | 2.084 |
| 19.806 | 4.479 | 31.871 | 2.805 | 46.77 | 1.941 |
| 20.10 | 4.414 | 32.67 | 2.738 | 47.71 | 1.905 |
| 20.481 | 4.333 | 33.13 | 2.702 | 49.53 | 1.839 |
| 21.818 | 4.070 | 33.869 | 2.645 | 50.67 | 1.800 |
| 22.486 | 3.951 | 33.641 | 2.662 | 53.50 | 1.711 |
| 23.757 | 3.742 | 34.746 | 2.579 | | |

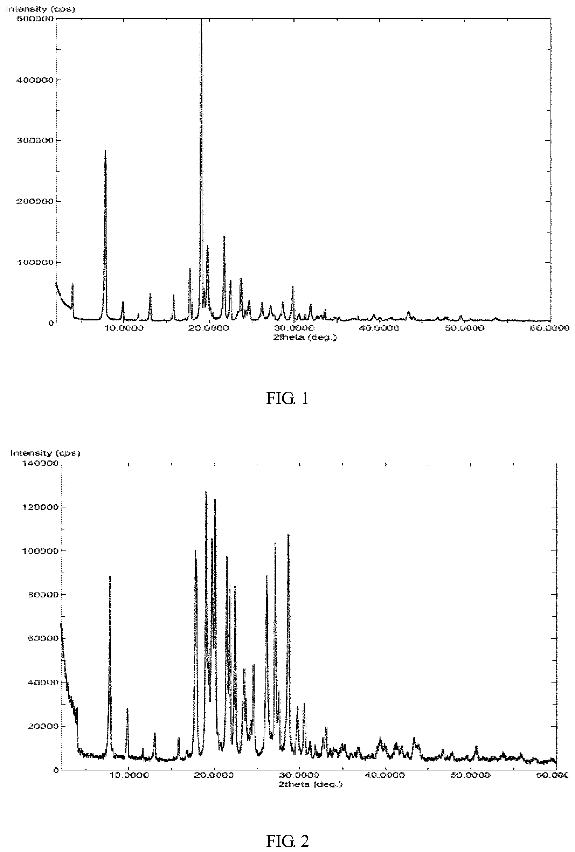

When analyzed with X-ray diffraction (CuKa), the X-ray diffraction pattern is as shown in FIG. 1.

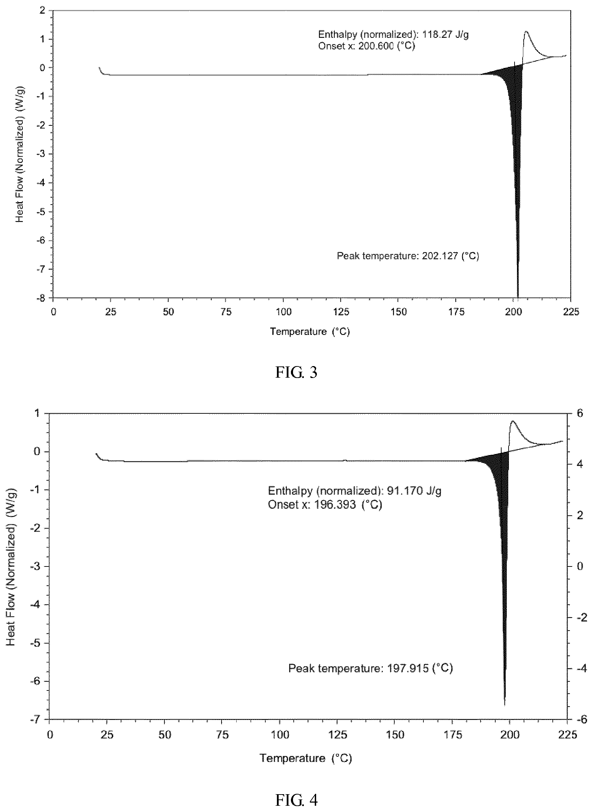

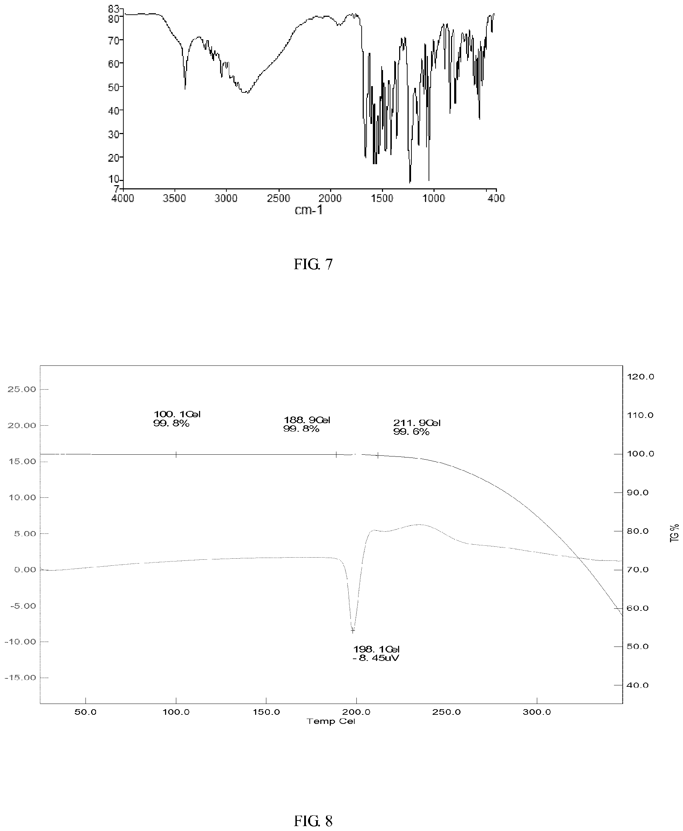

The Mod. I crystalline form has a melting point of 200.6 °C and its DSC thermoanalysis result is as shown in FIG. 3; TGA analysis shows that the Mod. I crystalline form has a degradation temperature of 241.71 °C and its TGA graph is as shown in FIG. 8.

Spectrum bands appear at 3415 cm⁻¹, 3058 cm⁻¹, 2805 cm⁻¹, 1668 cm⁻¹, 1652 cm⁻¹ and 1227 cm⁻¹ in the infrared spectrum (KBr) for the Mod. I crystalline form, and its infrared detection result is as shown in FIG. 7.

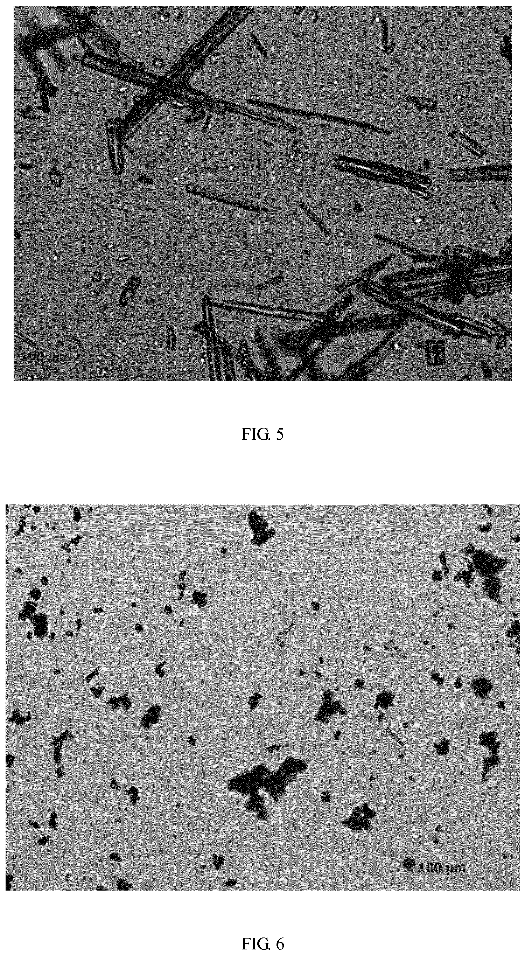

The result of hot-stage polarized optical microscope analysis of the Mod. I crystalline form shows that the product has an acicular crystal, having a relatively large particle length, which may be up to hundreds of microns, and have a high crystallinity. The HSM image is as shown in FIG. 5.

The present invention also provides a preparation method for the Mod. I crystalline form, comprising the following steps: dissolving a free alkali of EOC315 in an organic solvent, and adding a methanesulfonic acid in an organic solution dropwise; then a portion of the solvent is distilled off, ; and the EOC315 crystalline form Mod. I is obtained after adding another solvent for crystallization with temperature decrease.

The detailed preparation method for the Mod. I crystalline form is as follows:
1) dissolving a free alkali of EOC315 in a solvent to obtain an EOC315 solution, and dissolving a methanesulfonic acid in a solvent to obtain a methanesulfonic acid solution;
2) adding the methanesulfonic acid solution to the EOC315 solution at 20∼90 °C;
3) removing most of the solvent through vacuum distillation, and adding a polar or non-polar solvent, and reslurrying the solution at 0-30 °C, and collecting a filter cake after filtration;
4) vacuum drying the collected filter cake at 25-90 °C.

The solvent in step 1) may be a single organic solvent selected from methanol, ethanol, isopropanol, butanol, hexane, heptane, acetone, ethyl propyl ether, tetrahydrofuran, toluene, ethyl acetate and acetonitrile, or a mixture solvent thereof.

Preferably, the solvent in step 1) is a single solvent selected from methanol, ethanol, isopropanol and butanol, or a mixture solvent thereof.

Particle size of a drug affects the solubility of the drug (particularly for oral solid preparations) in various solvents, thereby directly affecting the bioavailability of the drug in a human body, which directly determines the efficacy of the drug.

The dried EOC315 crystalline form Mod. I is micronized to obtain the EOC315 crystalline form Mod. I micropowder, with a particle size D90 ≤ 15µm.

When analyzed with X-ray powder diffraction (CuKa), the EOC315 crystalline form Mod. I micropowder has an X-ray diffraction pattern as shown in FIG. 2, and is substantially consistent with the pattern prior to micronization, indicating that the crystalline form of the sample does not change after micronization, but the solid properties changed, for example, the size and preferred orientation of particles may affect the relative intensities of the peaks in the pattern, but do not affect the positions of the peaks, especially the positions of peaks with low 2Θ values. The pattern obtained after micronization also shows a certain degree of base line upheaval, which may indicate influences of crystallinity.

2Θ and d(A) of the X-ray diffraction pattern obtained after micronization are as shown in table 2.

**Table 2: 2Θ and d(A) values of the micronized EOC315 crystalline form Mod. I**

| 2Θ | d(A) | 2θ | d(A) | 2θ | d(A) |
|---|---|---|---|---|---|
| 3.898 | 22.65 | 23.413 | 3.796 | 35.209 | 2.547 |
| 7.766 | 11.375 | 23.616 | 3.764 | 36.74 | 2.444 |
| 9.856 | 8.967 | 24.23 | 3.670 | 39.38 | 2.286 |
| 12.953 | 6.829 | 24.521 | 3.627 | 39.92 | 2.256 |
| 15.777 | 5.613 | 26.09 | 3.413 | 41.11 | 2.194 |
| 17.798 | 4.979 | 27.07 | 3.291 | 41.46 | 2.176 |
| 18.998 | 4.667 | 27.534 | 3.237 | 41.91 | 2.154 |
| 19.357 | 4.582 | 28.608 | 3.118 | 43.34 | 2.086 |
| 19.734 | 4.475 | 29.715 | 3.004 | 46.708 | 1.943 |
| 20.039 | 4.427 | 30.480 | 2.930 | 47.838 | 1.899 |
| 21.423 | 4.144 | 31.80 | 2.812 | 50.61 | 1.802 |
| 21.742 | 4.084 | 32.66 | 2.740 | | |
| 22.394 | 3.967 | 33.05 | 2.708 | | |
| 23.27 | 3.820 | 33.869 | 2.645 | | |

The EOC315 crystalline form Mod. I micropowder has a transition temperature of 196.39 °C, which is lower than 200.6 °C (its transition temperature before the micronization), indicating that the micronized crystalline has a lower crystallinity, and the DSC thermoanalysis result of the micronized crystal is as shown in Fig 4.

Result of the hot-stage polarized optical microscope (HSM) analysis of the EOC315 crystalline form Mod. I micropowder shows that the sample is micropowder crystals (less than 10 microns) and has a lower crystallinity, and the HSM analysis result is as shown in FIG. 6.

According to the HSM analysis result, X-ray powder diffraction result and DSC result, EOC315 crystalline form Mod. I micropowder is of the crystalline form Mod. I.

The crystalline form of the EOC315 crystalline form Mod. I micropowder remains unchanged compared with that of the un-micronized compound, and both are the Mod. I crystalline form except that the un-micronized compound has a larger particle size and a higher purity.

In another aspect, the present invention further provides a pharmaceutical composition, comprising said EOC315 crystalline form Mod. I of the present invention and one or more pharmaceutically acceptable carriers or excipients. The EOC315 crystalline form Mod. I of the present invention and one or more pharmaceutically acceptable carriers or excipients may be formulated into a common dosage form in the pharmaceutical field, such as a tablet, a capsule, an injection, etc., and preferably an oral solid preparation. The EOC315 crystalline form Mod. I and one or more solid carriers may be mixed to prepare an orally-delivered pharmaceutical composition. The mixture may be granulated and the obtained granules may be mixed, when desired. Tablets or tablet cores may be obtained by adding an additional excipient, when desired or necessary. In particular, suitable carriers are filler (such as sugar (such as lactose, saccharose, mannitol or sorbitol), cellulose preparation and/or calcium phosphate (such as tricalcium phosphate or calcium hydrogen phosphate), and binder (for example starch (such as corn starch, wheat starch, rice starch or potato starch, methylcellulose, hydroxypropyl methylcellulose, sodium carboxymethylcellulose and/or polyvinylpyrrolidone) and/or disintegrator if needed (such as the aforementioned starch, carboxymethyl starch, cross-linked polyvinylpyrrolidone, alginic acid or salt thereof (such as sodium alginate)). In particular, additional excipients may be fluidity regulator and lubricant, such as silicic acid, talcum, stearic acid or a salt thereof (such as magnesium stearate or calcium stearate), and/or polyethylene glycol or derivatives thereof.

The tablet core may have an appropriate coating, and the coating is conducted by particularly using a concentrated sugar solution which can contain Arabic gum, talcum, polyvinylpyrrolidone, polyethylene glycol and/or titanium dioxide, or conducted in a coating solution in a suitable organic solvent or solvent mixture. Dye or pigment may be added into the tablet or the tablet coating, for example, for identifying or demonstrating the different doses of EOC315.

In another aspect, the present invention also provides the EOC315 crystalline form Mod. I as a potent VEGFR-2 (Vascular Endothelial Growth Factor Receptor-2) and PDGFR (Platelet-Derived Growth Factor Receptor) kinase inhibitor, which can effectively inhibit the growth of relevant tumor cells.

EOC315 may be used in combination with a standard therapeutic drug such as taxol (Taxol^{®}), capecitabine (Xeloda^{®}) and gemcitabine (Gemzar^{®}), which
1) produces better efficacy in decreasing the growth of a tumor or even eliminating the tumor compared with the delivery of any single agent aforementioned;
2) provides lower dosage of the chemotherapeutic agent administered;
3) provides a chemotherapeutic method that may be well-tolerated by a patient and has less observed harmful pharmacological complications than a mono-chemotherapy and other certain combination therapies;
4) provides a broader therapeutic spectrum for a different type of cancer in mammals, and particularly in human beings;
5) provides a higher speed of response for treated patients;
6) provides a longer survival time for the treated patients compared with standard chemotherapies;
7) provides a longer tumor progression time compared with another anticancer agent used in combination, which have caused antagonism , and/or produces at least the same efficacy and tolerance as the agent used alone.

EOC315 can be simultaneously, separately or consecutively delivered in a pharmaceutically effective amount with one or more of these cytotoxic agents. The dose of the delivered combined active agents ("pharmaceutically effective amount") varies in a broad range, depending on the to-be-treated disease and the delivery ways. The dose may comprise any amount that is effective to achieve an expected treatment. It is within the abilities of those skilled in the art to determine the "pharmaceutically effective amount" of the combined active agents.

### BRIEF DESCRIPTION OF THE DRAWINGS

FIG. 1 shows an X-ray powder diffraction pattern of an EOC315 crystalline form Mod. I;
FIG. 2 shows an X-ray diffraction pattern of the micronized EOC315 crystalline form Mod. I;
FIG. 3 shows a differential scanning calorimetry (DSC) graph of the EOC315 crystalline form Mod. I;
FIG. 4 shows a differential scanning calorimetry (DSC) graph of the micronized EOC315 crystalline form Mod. I;
FIG. 5 shows an image displaying a hot-stage polarized optical microscope (HSM) result of the EOC315 crystalline form Mod. I;
FIG. 6 shows an image displaying a hot-stage polarized optical microscope (HSM) result of the micronized EOC315 crystalline form Mod. I;
FIG. 7 shows an infrared (IR) spectrum of the EOC315 crystalline form Mod. I;
FIG. 8 shows a thermogravimetric analysis (TG/DTA) graph of the EOC315 crystalline form Mod. I.

### DETAILED DESCRIPTION

The present invention can be further described with the following examples. However, the present invention is not limited to the following examples, and these examples do not limit the scope of the present invention in any way. Certain changes and adjustments made by those skilled in the art within the scope of the claims shall be considered to fall within the scope of the present invention.

### Example 1: Preparation of EOC315 crystalline form Mod. I

The preparation process of an EOC315 crystalline form Mod. I comprised the following steps:
1) 9 g EOC315 alkali was dissolved in 90 mL methanol to obtain an EOC315 solution, and 2.5g methanesulfonic acid was dissolved in 16mL methanol to obtain a methanesulfonic acid solution;
2) the methanesulfonic acid solution was added dropwise to the EOC315 solution at 50 °C, and then was heated for 15 min;
3) a portion of the solvent was removed through the vacuum distillation conducted on the filtrate, then 200 ml isopropanol was added and vacuum distillation was continued until the solution volume was about 250 mL, then the solution was subjected to cooling crystallization for 1 hr, and the liquid was sucked away to collect the filter cake;
4) the collected filter cake was dried under vacuum at 45 °C to obtain EOC315 crystalline form Mod. I, with a yield of 90.5∼96.3% and a purity of 99.1∼99.9%.

The X-ray powder diffraction pattern as shown in FIG. 1 was obtained from the X-ray diffraction analysis.

### Example 2: Analysis of the EOC315 crystalline form Mod. I of example 1 by differential scanning calorimetry (DSC)

The DSC trace was recorded in an aluminum dish at the heating speed of 10 °C/min in a nitrogen atmosphere, and it was determined that there appeared a sharp endothermic peak at 200.7 °C for the EOC315 obtained in example 1, indicating that EOC315 was in a single Mod. I crystalline form with a melting point of 200.7 °C. The analysis result is as shown in FIG. 3.

As known by those of ordinary skilled in the art, the determined melting temperature depends on the experimental conditions, and particularly on the heating speed adopted. In addition, the melting temperature would be influenced by the substance purity. The reported melting temperature was determined by using the product with a purity of at least 98.5%.

### Example 3: Analysis of the EOC315 crystalline form Mod. I of example 1 by X-ray powder diffraction

Germanium-monochromatized CuKα1- radiation was used to record the X-ray powder diffraction data at room temperature. A small linear position-sensitive detector was used to carry out the 2θ scanning within a range of 3°≤2θ≤35° (step length: 0.5°) with an angular resolution of 0.08° at room temperature. The X-ray powder diffraction pattern is as shown in FIG. 1, wherein the values of 2θ and d(A) are as shown in table 1.

Those of ordinary skilled in the art will appreciate that there may be a measurement deviation in the obtained X-ray diffraction pattern, depending on the measurement conditions. In particular, it is generally known that the intensity of an X-ray diffraction pattern may fluctuate according to the crystal habit of a substance and the adopted measurement conditions. In addition, the measurement deviation of a diffraction angle θ of a conventional X-ray diffraction pattern is usually about ± 0.1° at a given temperature, and moreover, such degree of measurement deviation shall be taken into consideration when the diffraction angle is involved. Therefore, any crystalline form with an X-ray diffraction pattern consistent with the X-ray powder diffraction pattern disclosed in the figures shall fall within the scope of the present invention.

### Example 4: Thermogravimetric analysis (TG/DTA) of the EOC315 crystalline form Mod. I of example 1

Under a nitrogen atmosphere and at a heating speed of 10 °C/min, it was measured that EOC315 obtained in example 1 had a mass fraction of 99.8% at 188.9 °C, with a weight loss of 0.2%, nearly no weight loss, and this indicated that there was no crystallization solvent and a low content of volatile substances in the sample. The DTA curve of a test sample has an endothermic peak at 198.1 °C, which is consistent with the DSC result, and the TGA graph is as shown in FIG. 8.

As known by those of ordinary skill in the art, the measured temperature depends on the experimental conditions, and particularly on the heating speed adopted. In addition, the temperature would be influenced by the substance purity. The reported melting temperature was determined by using the batch with a purity of at least 98.5%.

### Example 5: Hot-stage polarized optical microscope (HSM) analysis of the EOC315 crystalline form Mod. I

The result of HSM analysis on an EOC315 sample showed that this sample was an acicular crystal with large granularity (with lengths up to hundreds of microns), and the crystallinity was high, and the HSM image is as shown in FIG. 5.

### Example 6: Infrared analysis of the EOC315 crystalline form Mod. I of example 1

Diffuse reflection (KBr) was used to record the infrared spectrum of the EOC315 Mod. I crystal. The infrared spectrum is as shown in FIG. 7, and the main infrared bands and assignments thereof are as shown in table 3.

**Table 3: Main absorption peaks in the infrared spectrum of the EOC315 Mod. I crystal and assignments thereof**

| Sample No. | Absorption peaks of test samples (cm⁻¹) | Vibration type | Group (assignment) | Intensities of absorption peaks |
|---|---|---|---|---|
| 1 | 3414.66 | V_{N-H} | N-H stretching vibration | m |
| 2 | 3057.73 | V_{C-H} | C-H stretching vibration in aromatic ring | m |
| 3 | 2804.69 | V_{C-H} | Saturated C - H stretching vibration | m |
| 4 | 1668.18 | V_{C=O} | C=O stretching vibration | vs |
| 5 | 1652.40, 1608.18, 1584.45, 1557.03, 1531.35, 1492.12 | V_{C-C}, V_{C-N} | Skeleton stretching vibration of aromatic ring | m∼s |
| 6 | 1227.16, 1142.23, 1067.69, 1040.92 | V_{C-O}, V_{C-N} | C-O and C-N stretching vibrations | vs |

As known by those of ordinary skilled in the art, the acceptable wavenumber shift tolerance is ±2 cm⁻¹ based on the adopted instruments and measurement conditions. Therefore, any solid-state form with an FT-Raman spectrum consistent with the FT-Raman spectrum disclosed in the drawing may fall within the scope of the present invention.

### Example 7: Micronization of the EOC315 crystalline form Mod. I obtained in Example 1

The pressure of the micronization equipment was set to be ≥0.65 MPa to obtain an EOC315 crystalline form micropowder, with a particle size D90 ≤15 µm. The X-ray powder diffraction, DSC and HSM data of the micronized sample were measured by the aforementioned methods, and the results are as shown in FIG. 2, FIG. 4 and FIG. 6.

It can be concluded from the results shown in the drawings that the crystalline form of the micronized EOC315 remained unchanged, and was still the crystalline form Mod. I.

### Example 8: Inhibitory effect of the EOC315 crystalline form Mod. I on tumor cells

The EOC315 crystalline form Mod. I shows biochemical and cellular activity in vitro, and is consistent with the anti-angiogenesis action mechanism. Therefore, a study was conducted in a Colo-205 human CRC xenograft model to evaluate whether the anti-tumor activity observed in vivois consistent with the hypothesis. The microvessel density of a tumor was determined by the quantitative histomorphometry after the endothelial cell marker CD31 was stained. 4 hours after the first drug delivery, the declining level of the tumor MVA was further increased compared with each solvent control group (32%; P <0.05). 24 hours after the administration, the declining level of the tumor MVA was further increased (53%; P < 0.01). The effect was more obvious 3 days after the administration, and moreover, MVA was observed to decrease by 64% and 68% at 4 hours and 6 hours respectively.

Those skilled in the art may discover from the aforementioned experimental results that the growth of tumor cells was significantly suppressed when an EOC315 crystalline form Mod. I was used.

### Example 9: Study on the efficacy of EOC315 used in combination with capecitabine (X) and cisplatinum (P) in patients with advanced gastric cancers or gastroesophageal junction (GEJ) cancers

After at most 6 cycles of cisplatinum treatment, both or either of capecitabine and EOC315 was delivered to a subject in a previous dosage according to the toxic reaction conditions, until the disease progressed.

48 subjects in total were enrolled for this study. 45 subjects (94%) had tumor metastasis and 35 subjects (73%) had more than two metastatic sites thereinto. The livers of 26 subjects (54%) were involved due to the tumors. The major sites involved for the 26 subjects (54%) were gastroesophageal junctions, the major sites involved for 22 subjects (46%) were stomach, and the majority of the subjects got an ECOG score of 1 (n=41, 85%).

39 subjects completed at least one course of treatment and got therapeutic effect evaluation. The objective response rate (ORR) was 67%. One subject (2.6%) achieved the complete response, and 25 subjects (64.1%) achieved the partial response. 11 subjects (28.2%) showed stable conditions and maintained for more than 12 weeks. It was evaluated that the objective response generally occurred in the previous two cycles of treatment and lasted for a long time, which was related closely with the duration of treatment received by the subjects. The anti-tumor effect was not related to tumor tissue type, the primary site, whether or not liver is involved, or the study center. Median progression-free survival (PFS) was 4.5 months. Kaplan-Meier analysis showed that the proportion of patients without tumor-progression within 6 months was 52%. The median time to progression for patients showing tumor remission was 6.4 months. Median OS was estimated to be 7.8 months, and the 1-year survival rate was 33%.

The preliminary result showed a high overall response rate (67% of objective response rate (ORR), which was almost twice the historical response rate achieved by chemotherapy alone) and a long response time, and there was evidence showing a prolonged survival.

### Example 10: Result of the stability experiment

12 month long-term stability was evaluated under 25 °C, with a relative humidity of 60%; or 6 month accelerated stability was evaluated under 40 °C, with a relative humidity of 75%, and the results are as shown in table 4 and 5 below:

**Table 4: The long-term stability study of EOC315 (temperature: 25°C ± 2°C; relative humidity (RH): 60% ± 5%)**

| Item evaluated | Limit requirement | | Batch No. | Month 0 | | Month 3 | | Month 6 | | Month 9 | | Month 12 | |
|---|---|---|---|---|---|---|---|---|---|---|---|---|---|
| Related substance | Impurity 1 | 0.17% | 151211 | Impurity 1 | Undetected | Impurity 1 | Undetected | Impurity 1 | Undetecte d | Impurity 1 | Undetected | Impurity 1 | Undetected |
| | | 0.17% | | | | | | | | | | | |
| | Impurity 2 | 0.17% | | Impurity 2 | Undetected | Impurity 2 | Undetected | Impurity 2 | 0.0028% | Impurity 2 | Undetected | Impurity 2 | Undetected |
| | | 3.0% | | | | | | | | | | | |
| | Impurity 3 | 0.10% % | | Impurity 3 | 0.016% | Impurity 3 | Undetected | Impurity 3 | 0.099% | Impurity 3 | Undetected | Impurity 3 | Undetected |
| | Impurity 4 | 0.10% | | Impurity 4 | 0.36% | Impurity 4 | 0.4% | Impurity 4 | 0.42% | Impurity 4 | 0.43% | Impurity 4 | 0.46% |
| | Impurity 5 | 0.15% | | Impurity 5 | 0.02% | Impurity 5 | 0.01% | Impurity 5 | Undetecte d | Impurity 5 | 0.02% | Impurity 5 | 0.01% |
| | Impurity 6 | 1.0% | | Impurity 6 | 0.01% | Impurity 6 | Undetected | Impurity 6 | 0.01% | Impurity 6 | Undetected | Impurity 6 | 0.03% |
| | Maximum single impurity | | | Maximu m single impurity | 0.0079% | Maximu m single impurity | Undetected | Maximu m single impurity | 0.013% | Maximum single impurity | 0.013% | Maximum single impurity | Undetected |
| | Total impurity | | | Total impurity | 0.53% | Total impurity | 0.5% | Total impurity | 0.73% | Total impurity | 0.61% | Total impurity | 0.63% |
| | | | | Impurity 1 | Undetected | Impurity 1 | Undetected | Impurity 1 | Undetecte d | Impurity 1 | Undetected | Impurity 1 | Undetected |
| | | | | Impurity 2 | Undetected | Impurity 2 | Undetected | Impurity 2 | Undetecte d | Impurity 2 | Undetected | Impurity 2 | Undetected |
| | | | | Impurity 3 | 0.011% | Impurity 3 | Undetected | Impurity 3 | 0.031% | Impurity 3 | Undetected | Impurity 3 | Undetected |
| | | | 151212 | Impurity 4 | 0.46% | Impurity 4 | 0.5% | Impurity 4 | 0.54% | Impurity 4 | 0.56% | Impurity 4 | 0.58% |
| | | | | Impurity 5 | 0.01% | Impurity 5 | 0.02% | Impurity 5 | 0.02% | Impurity 5 | 0.01% | Impurity 5 | Undetected |
| | | | | Impurity 6 | Undetected | Impurity 6 | Undetected | Impurity 6 | 0.01% | Impurity 6 | 0.01% | Impurity 6 | 0.02% |
| | | | | Maximu m single impurity | 0.012% | Maximu m single impurity | Undetected | Maximu m single impurity | 0.070% | Maximum single impurity | Undetected | Maximum single impurity | Undetected |
| | | | | Total impurity | 0.65% | Total impurity | 0.6% | Total impurity | 0.88% | Total impurity | 0.77% | Total impurity | 0.80% |
| | | | | Impurity 1 | Undetected | Impurity 1 | Undetected | Impurity 1 | Undetecte d | Impurity 1 | Undetected | Impurity 1 | Undetected |
| | | | | Impurity 2 | Undetected | Impurity 2 | Undetected | Impurity 2 | Undetecte d | Impurity 2 | Undetected | Impurity 2 | Undetected |
| | | | 151213 | Impurity 3 | 0.011% | Impurity 3 | Undetected | Impurity 3 | 0.033% | Impurity 3 | Undetected | Impurity 3 | Undetected |
| | | | | Impurity 4 | 0.35% | Impurity 4 | 0.38% | Impurity 4 | 0.40% | Impurity 4 | 0.42% | Impurity 4 | 0.42% |
| | | | | Impurity 5 | Undetected | Impurity 5 | 0.01% | Impurity 5 | Undetecte d | Impurity 5 | Undetected | Impurity 5 | 0.01% |
| | | | | Impurity 6 | Undetected | Impurity 6 | Undetected | Impurity 6 | 0.01% | Impurity 6 | Undetected | Impurity 6 | Undetected |

| | | | | Maximu m single impurity | 0.0085% | Maximu m single impurity | Undetected | Maximu m single impurity | 0.016% | Maximum single impurity | Undetected | Maximum single impurity | Undetected |
|---|---|---|---|---|---|---|---|---|---|---|---|---|---|
| | | | | Total impurity | 0.52% | Total impurity | 0.5% | Total impurity | 0.63% | Total impurity | 0.58% | Total impurity | 0.58% |
| Crystalline form | ModI | | 151211 | ModI | | / | | ModI | | / | | ModI | |
| | | | 15121 2 | ModI | | / | | ModI | | / | | ModI | |
| | | | 15121 3 | ModI | | / | | ModI | | / | | ModI | |

**Table 5: The stability evaluation of EOC315 under an accelerated condition (temperature: 40°C ± 2°C; relative humidity (RH): 75% ± 5%)**

| Study item | Limit requirement | | Batch No. | Month 0 | | Month 1 | | Month 2 | | Month 3 | | Month 6 | |
|---|---|---|---|---|---|---|---|---|---|---|---|---|---|
| Related substance | | | 151211 | Impurity 1 | Undetected | Impurity 1 | Undetected | Impurity 1 | Undetected | Impurity 1 | Undetect ed | Impurity 1 | Undetecte d |
| | | | | Impurity 2 | Undetected | Impurity 2 | 0.0031% | Impurity 2 | Undetected | Impurity 2 | Undetect ed | Impurity 2 | 0.0061% |
| | Impurity 1 | | | Impurity 3 | 0.016% | Impurity 3 | 0.0040% | Impurity 3 | 0.0013% | Impurity 3 | Undetect ed | Impurity 3 | 0.045% |
| | Impurity 2 | 0.17% | | Impurity 4 | 0.36% | Impurity 4 | 0.4% | Impurity 4 | 0.4% | Impurity 4 | 0.4% | Impurity 4 | 0.4% |
| | Impurity 3 | 0.17% | | Impurity 5 | 0.02% | Impurity 5 | Undetected | Impurity 5 | Undetected | Impurity 5 | 0.01% | Impurity 5 | 0.01% |
| | | 0.17% | | | | | | | | | | | |
| | Impurity 4 | 3.0% | | Impurity 6 | 0.01% | Impurity 6 | 0.01% | Impurity 6 | 0.01% | Impurity 6 | 0.02% | Impurity 6 | 0.01% |
| | | 0.10% | | | | | | | | | | | |
| | Impurity 5 | 0.10% | | Maximum single impurity | 0.0079% | Maximum single impurity | 0.011% | Maximum single impurity | 0.0045% | Maximum single impurity | Undetect ed | Maximum single impurity | 0.030% |
| | Impurity 6 | | | | | | | | | | | | |
| | | 0.15% | | Total impurity | 0.53% | Total impurity | 0.5% | Total impurity | 0.6% | Total impurity | 0.5% | Total impurity | 0.7% |
| | Maximum single impurity | | | | | | | | | | | | |
| | | 1.0% | 151212 | Impurity 1 | Undetected | Impurity 1 | Undetected | Impurity 1 | Undetected | Impurity 1 | Undetect ed | Impurity 1 | Undetecte d |
| | Total impurity | | | Impurity 2 | Undetected | Impurity 2 | 0.0026% | Impurity 2 | 0.0036% | Impurity 2 | Undetect ed | Impurity 2 | 0.0043% |
| | | | | Impurity 3 | 0.011% | Impurity 3 | 0.0037% | Impurity 3 | 0.0070% | Impurity 3 | Undetect ed | Impurity 3 | 0.044% |
| | | | | Impurity 4 | 0.46% | Impurity 4 | 0.5% | Impurity 4 | 0.5% | Impurity 4 | 0.5% | Impurity 4 | 0.5% |
| | | | | Impurity 5 | 0.01% | Impurity 5 | 0.01% | Impurity 5 | 0.02% | Impurity 5 | 0.01% | Impurity 5 | 0.01% |
| | | | | Impurity 6 | Undetected | Impurity 6 | Undetected | Impurity 6 | Undetected | Impurity 6 | Undetect ed | Impurity 6 | Undetecte d |
| | | | | Maximum single impurity | 0.012% | Maximum single impurity | Undetected | Maximum single impurity | 0.0062% | Maximum single impurity | Undetect ed | Maximum single impurity | 0.032% |
| | | | | Total impurity | 0.66% | Total impurity | 0.6% | Total impurity | 0.7% | Total impurity | 0.6% | Total impurity | 0.8% |
| | | | 151213 | Impurity 1 | Undetected | Impurity 1 | Undetected | Impurity 1 | Undetected | Impurity 1 | Undetect ed | Impurity 1 | Undetecte d |
| | | | | Impurity 2 | Undetected | Impurity 2 | Undetected | Impurity 2 | 0.0035% | Impurity 2 | Undetect ed | Impurity 2 | 0.045% |
| | | | | Impurity 3 | 0.011% | Impurity 3 | 0.0035% | Impurity 3 | 0.014% | Impurity 3 | Undetect ed | Impurity 3 | 0.060% |
| | | | | Impurity 4 | 0.35% | Impurity 4 | 0.3% | Impurity 4 | 0.4% | Impurity 4 | 0.37% | Impurity 4 | 0.4% |
| | | | | Impurity 5 | Undetected | Impurity 5 | 0.01% | Impurity 5 | Undetected | Impurity 5 | Undetect ed | Impurity 5 | Undetecte d |
| | | | | Impurity 6 | Undetected | Impurity 6 | Undetected | Impurity 6 | Undetected | Impurity 6 | 0.01%d | Impurity 6 | Undetecte d |
| | | | | Maximum single impurity | 0.0085% | Maximum single impurity | 0.0077% | Maximum single impurity | 0.0067% | Maximum single impurity | Undetect ed | Maximum single impurity | 0.029% |
| | | | | Total impurity | 0.52% | Total impurity | 0.5% | Total impurity | 0.6% | Total impurity | 0.5% | Total impurity | 0.7% |
| Crystalline form | ModI | | 151211 | ModI | | / | | / | | / | | ModI | |
| | | | 151212 | ModI | | / | | / | | / | | ModI | |
| | | | 151213 | ModI | | / | | / | | / | | ModI | |

It can be concluded from the above tables that the Crystalline form Mod. I of EOC315 is a thermodynamically stable form.

The produced drug was micronized to increase its bioavailability, and the crystalline form of the micronized drug was still the Mod. I crystalline form except that its crystallinity was decreased.

## Claims

1. The crystalline form Mod. I of Formula I, 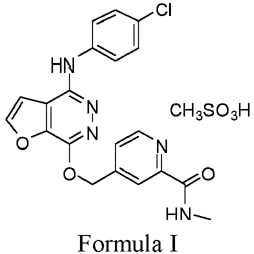 which has an X-ray powder diffraction diagram with peaks at 4.01° (±0.1°), 7.85° (±0.1°), 9.94° (±0.1°), 13.04° (±0.1°), 19.08° (±0.1°), 19.46° (±0.1°), 20.10° (±0.1°), 21.82° (±0.1°), 22.49° (±0.1°), 23.76° (±0.1°), 24.26° (±0.1°), 27.17° (±0.1°), 28.52° (±0.1°) and 30.48° (±0.1°) 2θ, when analyzed with X-ray diffraction CuKa.

2. The crystalline form of claim 1, wherein said crystalline form Mod. I has an X-ray powder diffraction diagram the same as the X-ray powder diffraction diagram shown in FIG. 1 when analyzed with the X-ray powder diffraction CuKa.

3. The crystalline form of any one of claims 1 to 2, wherein said crystalline form Mod. I has a spectrum band at 3415 cm⁻¹ (±2 cm⁻¹), 3058 cm⁻¹ (±2 cm⁻¹), 2805 cm⁻¹ (±2 cm⁻¹), 1668 cm⁻¹ (±2 cm⁻¹), 1652 cm⁻¹ (±2 cm⁻¹) and 1227 cm⁻¹ (±2 cm⁻¹), when analyzed with an infrared spectroscopy KBr.

4. The crystalline form of any one of claims 1 to 3, wherein said crystalline form Mod. I has an infrared spectrum the same as the infrared spectrum shown in FIG. 7

5. The crystalline form of any one of claims 1 to 4, wherein said crystalline form Mod. I has a melting point of about 200.6°C.

6. A micropowder of the crystalline form Mod. I of Formula I, defined in claim 1, 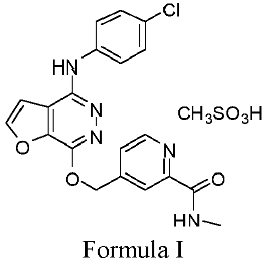 which has an X-ray powder diffraction diagram the same as the X-ray powder diffraction diagram shown in FIG. 2 when analyzed with X-ray powder diffraction CuKa, and wherein said micropowder has a particle size D90 ≤ 15 µm.

7. A process for the preparation of a crystalline form as claimed in any one of claims 1 to 5, comprising the following steps:
1) dissolving the 4-(4-chloroanilino)-7-(2-methylaminocarbonyl-4-oxymethyl) pyridylfuro[2,3-d]pyridazine mesylate in a solvent to obtain a solution of the compound, and dissolving a methanesulfonic acid in a solvent to obtain a methanesulfonic acid solution;
2) adding the methanesulfonic acid solution to the solution of the compound at 20 to 90°C;
3) removing most of the solvent through vacuum distillation, adding a polar or non-polar solvent, reslurrying at 0-30°C, and collecting a filter cake after filtration;
4) vacuum drying the collected filter cake at 25-90°C.

8. A pharmaceutical composition comprising the crystalline form of any one of claims 1 to 5 and/or a micropowder according to claim 6, and a pharmaceutically acceptable carrier or excipient.

9. A crystalline form of any one of claims 1 to 5 and/or a micropowder of claim 6, wherein the crystalline and/or the micropowder is for use in treating cancer in a subject in need thereof.

10. The crystalline form and/or the micropowder for the use according to claim 9, wherein said cancer comprises an advanced solid tumor.

11. The crystalline form and/or the micropowder for the use according to claim 9, wherein said cancer is selected from the group consisting of a gastric cancer and a gastroesophageal junction (GEJ) cancer.

12. The crystalline form and/or the micropowder for the use according to any one of claims 9 to 11, wherein said subject is further treated with one or more additional chemotherapeutic agent.

13. The crystalline form and/or the micropowder for the use according to claim 12, wherein said one or more additional chemotherapeutic agent is selected from the group consisting of taxol, capecitabine, cisplatin and gemcitabine.

14. The crystalline form and/or the micropowder for the use according to any one of claims 9 to 11, wherein said subject is further treated with capecitabine and cisplatin.

## Patentansprüche

1. Kristalline Form Mod. I der Formel I, 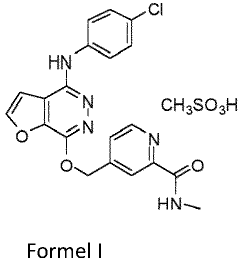 die bei Analyse mit CuKa Röntgendiffraktion ein Röntgenpulverdiffraktionsdiagramm mit Peaks bei 4,01° (± 0,1°), 7,85° (± 0,1°), 9,94° (± 0,1°), 13,04° (± 0,1°), 19,08° (± 0,1°), 19,46° (± 0,1°), 20,10° (± 0,1°), 21,82° (± 0,1°), 22,49° (± 0,1°), 23,76° (± 0,1°), 24,26° (± 0,1°), 27,17° (± 0,1°), 28,52° (± 0,1°) und 30,48° (± 0,1°) 2θ aufweist.

2. Kristalline Form nach Anspruch 1, wobei die kristalline Form Mod. I bei Analyse mit CuKa Röntgenpulverdiffraktion dasselbe Röntgenpulverdiffraktionsdiagramm aufweist, wie das in Fig. 1 gezeigte Röntgenpulverdiffraktionsdiagramm.

3. Kristalline Form nach einem der Ansprüche 1 bis 2, wobei die kristalline Form Mod. I bei Analyse mit KBr Infrarotspektroskopie eine Spektralbande bei 3415 cm⁻¹ (± 2 cm⁻¹), 3058 cm⁻¹ (± 2 cm⁻¹), 2805 cm⁻¹ (± 2 cm⁻¹), 1668 cm⁻¹ (± 2 cm⁻¹), 1652 cm⁻¹ (± 2 cm⁻¹) und 1227 cm⁻¹ (± 2 cm⁻¹) aufweist.

4. Kristalline Form nach einem der Ansprüche 1 bis 3, wobei die kristalline Form Mod. I dasselbe Infrarotspektrum aufweist, wie das in Fig. 7 gezeigte Infrarotspektrum.

5. Kristalline Form nach einem der Ansprüche 1 bis 4, wobei die kristalline Form Mod. I einen Schmelzpunkt von ungefähr 200,6°C aufweist.

6. Mikropulver der kristallinen Form Mod. I der Formel I, definiert in Anspruch 1, 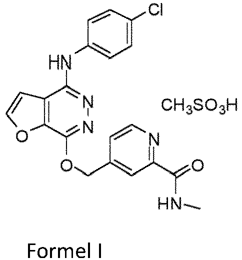 das bei Analyse mit CuKa Röntgenpulverdiffraktion dasselbe Röntgenpulverdiffraktionsdiagramm aufweist, wie das in Fig. 2 gezeigte Röntgenpulverdiffraktionsdiagramm und wobei das Mikropulver eine Partikelgröße D90 ≤ 15 µm aufweist.

7. Verfahren zur Herstellung einer kristallinen Form nach einem der Ansprüche 1 bis 5, umfassend die folgenden Schritte:
1) Lösen des 4-(4-Chloranilin)-7-(2-methylaminocarbonyl-4-oxymethyl)pyridylfuro[2,3-d]pyridazinmesylats in einem Lösungsmittel, um eine Lösung der Verbindung zu erhalten, und Lösen einer Methansulfonsäure in einem Lösungsmittel, um eine Methansulfonsäurelösung zu erhalten;
2) Zugeben der Methansulfonsäurelösung zu der Lösung der Verbindung bei 20 bis 90°C;
3) Entfernen des größten Teils des Lösungsmittels durch Vakuumdestillation, Zugeben eines polaren oder unpolaren Lösungsmittels, Wiederaufschlämmen bei 0-30°C und Sammeln eines Filterkuchens nach Filtration;
4) Vakuumtrocknen des gesammelten Filterkuchens bei 25-90°C.

8. Pharmazeutische Zusammensetzung, umfassend die kristalline Form nach einem der Ansprüche 1 bis 5 und/oder ein Mikropulver nach Anspruch 6 und einen pharmazeutisch verträglichen Träger oder Hilfsstoffe.

9. Kristalline Form nach einem der Ansprüche 1 bis 5 und/oder ein Mikropulver nach Anspruch 6, wobei die kristalline Form und/oder das Mikropulver zur Verwendung bei der Behandlung von Krebs in einem Subjekt, das dies benötigt, ist.

10. Kristalline Form und/oder Mikropulver zur Verwendung nach Anspruch 9, wobei der Krebs einen fortgeschrittenen soliden Tumor umfasst.

11. Kristalline Form und/oder Mikropulver zur Verwendung nach Anspruch 9, wobei der Krebs ausgewählt ist aus der Gruppe bestehend aus Magenkrebs und einem Krebs der gastroösophagealen Verbindung (GEJ).

12. Kristalline Form und/oder Mikropulver zur Verwendung nach einem der Ansprüche 9 bis 11, wobei das Subjekt ferner mit einem oder mehreren zusätzlichen chemotherapeutischen Mitteln behandelt wird.

13. Kristalline Form und/oder Mikropulver zur Verwendung nach Anspruch 12, wobei das eine oder die mehreren zusätzlichen chemotherapeutischen Mitteln ausgewählt sind aus der Gruppe bestehend aus Taxol, Capecitabin, Cisplatin und Gemcitabin.

14. Kristalline Form und/oder Mikropulver zur Verwendung nach einem der Ansprüche 9 bis 11, wobei das Subjekt ferner mit Capecitabin und Cisplatin behandelt wird.

## Revendications

1. Forme cristalline Mod.I de la formule I, 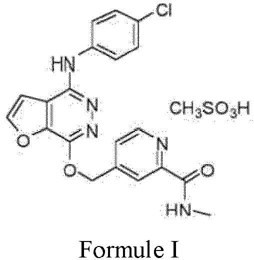 qui a un diagramme de diffraction des rayons X sur poudre avec des pics à 4,01° (±0,1°), 7,85° (±0,1°), 9,94° (±0,1°), 13,04° (±0,1°), 19,08° (±0,1°), 19,46° (±0,1°), 20,10° (±0,1°), 21,82° (±0,1°), 22,49° (±0,1°), 23,76° (±0,1°), 24,26° (±0,1°), 27,17° (±0,1°), 28,52° (±0,1°) et 30,48° (±0,1°) 2θ, lorsqu'elle est analysée avec la diffraction des rayons X CuKa.

2. Forme cristalline selon la revendication 1, dans laquelle ladite forme cristalline Mod.I a un diagramme de diffraction des rayons X sur poudre identique au diagramme de diffraction des rayons X sur poudre représenté dans la FIG. 1, lorsqu'elle est analysée avec la diffraction des rayons X sur poudre CuKa.

3. Forme cristalline selon l'une quelconque des revendications 1 et 2, dans laquelle ladite forme cristalline Mod.I a une bande spectrale à 3415 cm⁻¹ (±2 cm⁻¹), 3058 cm⁻¹ (±2 cm⁻¹), 2805 cm⁻¹ (±2 cm⁻¹), 1668 cm⁻¹ (±2 cm⁻¹), 1652 cm⁻¹ (±2 cm⁻¹) et 1227 cm⁻¹ (±2 cm⁻¹), lorsqu'elle est analysée avec une spectroscopie infrarouge KBr.

4. Forme cristalline selon l'une quelconque des revendications 1 à 3, dans laquelle ladite forme cristalline Mod.I a un spectre infrarouge identique au spectre infrarouge représenté dans la FIG. 7.

5. Forme cristalline selon l'une quelconque des revendications 1 à 4, dans laquelle ladite forme cristalline Mod.I a un point de fusion d'environ 200,6 °C.

6. Micropoudre de la forme cristalline Mod.I de la Formule I, définie dans la revendication 1, 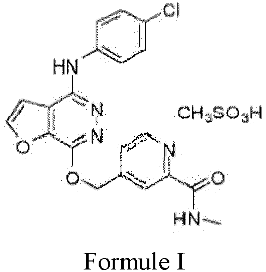 qui a un diagramme de diffraction des rayons X sur poudre identique au diagramme de diffraction des rayons X sur poudre représenté dans la FIG. 2, lorsqu'elle est analysée avec une diffraction des rayons X sur poudre CuKa, et dans laquelle ladite micropoudre a une taille de particule D90 ≤ 15 µm.

7. Processus de préparation d'une forme cristalline selon l'une quelconque des revendications 1 à 5, comprenant les étapes suivantes :
1) dissoudre le 4-(4-chloroanilino)-7-(2-méthylaminocarbonyl-4-oxyméthyl) pyridylfuro[2,3-d]pyridazine mésylate dans un solvant pour obtenir une solution du composé, et dissoudre un acide méthanesulfonique dans un solvant pour obtenir une solution d'acide méthanesulfonique ;
2) ajouter la solution d'acide méthanesulfonique à la solution du composé à une température de 20 à 90 °C ;
3) éliminer la majeure partie du solvant par distillation sous vide, ajouter un solvant polaire ou apolaire, remettre en suspension à une température de 0 à 30 °C, et recueillir un gâteau de filtration après filtration ;
4) sécher sous vide le gâteau de filtration recueilli à une température de 25 à 90 °C.

8. Composition pharmaceutique comprenant la forme cristalline selon l'une quelconque des revendications 1 à 5 et/ou une micropoudre selon la revendication 6, et un support ou excipient pharmaceutiquement acceptable.

9. Forme cristalline selon l'une quelconque des revendications 1 à 5 et/ou micropoudre selon la revendication 6, dans lesquelles la forme cristalline et/ou la micropoudre est destinée à être utilisée dans le traitement du cancer chez un sujet qui en a besoin.

10. Forme cristalline et/ou micropoudre pour l'utilisation selon la revendication 9, dans lesquelles ledit cancer comprend une tumeur solide avancée.

11. Forme cristalline et/ou micropoudre pour l'utilisation selon la revendication 9, dans lesquelles ledit cancer est choisi dans le groupe constitué d'un cancer gastrique et d'un cancer de la jonction gastro-œsophagienne (GEJ).

12. Forme cristalline et/ou micropoudre pour l'utilisation selon l'une quelconque des revendications 9 à 11, dans lesquelles ledit sujet est en outre traité avec un ou plusieurs agents chimiothérapeutiques supplémentaires.

13. Forme cristalline et/ou micropoudre pour l'utilisation selon la revendication 12, dans lesquelles lesdits un ou plusieurs agents chimiothérapeutiques supplémentaires sont choisis dans le groupe constitué du taxol, de la capécitabine, du cisplatine et de la gemcitabine.

14. Forme cristalline et/ou micropoudre pour l'utilisation selon l'une quelconque des revendications 9 à 11, dans lesquelles ledit sujet est en outre traité avec la capécitabine et le cisplatine.
